# EUROPÄISCHE PATENTSCHRIFT

(11) **EP 1 481 964 B1**
(45) Veröffentlichungstag und Bekanntmachung des Hinweises auf die Patenterteilung: **23.08.2006**
(21) Anmeldenummer: 04018487.1
(22) Anmeldetag: 15.11.2000
(51) Int. Cl.: C07C 215/54, C07C 219/28

(54) **Stabile Salze neuartiger derivative von 3,3-Diphenylpropylaminen**
Stable salts of novel derivatives of 3,3-diphenylpropylamines
Sels stabiles de nouveaux dérivés de 3,3-diphénylpropylamines

(30) Priorität: 16.11.1999 DE 19955190
(43) Veröffentlichungstag der Anmeldung: 01.12.2004
(62) Teilanmeldung aus: 00989857.8
(73) Patentinhaber: SCHWARZ PHARMA AG, 40789 Monheim/Rhld. (DE)
(72) Erfinder: Meese, Claus, 40789 Monheim (DE)
(74) Vertreter: Bausch, Thorsten

(56) Entgegenhaltungen:
- WO-A-94/11337
- WO-A-98/43942
- PALMER, L. ET AL.: "Determination of tolterodine and the 5-hydroxymetylmetabolite ..." J. PHARM. BIOMED. ANAL., Bd. 16, 1997, Seiten 155-165, XP000995770

## Beschreibung

Die vorliegende Erfindung betrifft die Verwendung bestimmter Verbindungen als Zwischenprodukte bei der Herstellung von kristallinem *R*-(+)-2-(3-Diisopropylamino-1-phenylpropyl)-4-hydroxymethyl-phenylisobuttersäureester Hydrogenfumarat, sowie letztere Verbindung in kristalliner Form.

Aus dem Dokument PCT/EP99/03212 sind neuartige Derivate von 3,3-Diphenylpropylaminen bekannt.

Sie sind wertvolle Prodrugs für die Behandlung von Harndrang-Inkontinenz und anderen spasmogenen Leiden, die den Nachteil bisher zur Verfügung stehender Wirkstoffe, nämlich zu geringe Absorption der Wirkstoffe durch biologische Membranen oder deren ungünstigen Metabolismus vermeiden.

Weiterhin zeichnen sich diese neuartigen Prodrugs durch verbesserte pharmakokinetische Eigenschaften im Vergleich zu Oxybutynin und Tolterodin aus.

Bevorzugte Verbindungen aus der Gruppe dieser neuartigen Derivate von 3,3-Diphenylpropylaminen sind Ester aliphatischer oder aromatischer Carbonsäuren mit der nachfolgend genannten allgemeinen Formel A 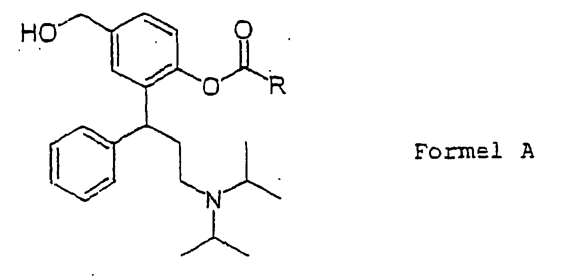 in der R die Bedeutung von C₁-C₆-alkyl, C₃-C₁₀-cycloalkyl oder unsubstituiertem oder substituiertem Phenyl hat. Sie können in Form ihrer optischen Isomere, als Racematengemisch und in Form ihrer individuellen Enantiomere vorliegen.

Verbindungen der Struktur der Formel A besitzen allerdings eine geringe Wasserlöslichkeit. Diese verringert ihre orale Bioverfügbarkeit.

Schließlich neigen Monoester der Struktur, wie sie in Formel A wiedergegeben sind, zu intermolekularer Umesterung.

Bei längerer Lagerung ist deshalb unter Gehaltsabnahme von Verbindungen der Struktur der allgemeinen Formel A eine Zunahme von Diester und freiem Diol feststellbar.

Zwar lassen sich grundsätzlich Salze der Verbindungen der allgemeinen Formel A erhalten, indem Lösungen der Verbindungen der Formel A (Basenteil) mit Lösungen von Säuren in jeweils geeigneten Lösungsmitteln vereinigt werden, jedoch erweisen sich die als Festkörper erhaltenen Salze als durchweg amorph und/oder hygroskopisch und sind auch aus den üblichen Lösungsmitteln nicht ohne weiteres kristallisierbar. Derartige Salze weisen eine zu geringe chemische Stabilität auf, um als wertvolle pharmazeutische Wirkstoffe galenisch verarbeitet werden zu können.

Es wurde gefunden, dass sich die vorgenannten Nachteile vermeiden lassen, wenn Verbindungen der Struktur der allgemeinen Formel A, nachdem sie unter spezieller Reaktionsführung dargestellt wurden, mit einer physiologisch verträglichen anorganischen oder organischen Säure der allgemeinen Formel H-X, in der X den jeweiligen Säurerest bedeutet, zu ihrem jeweiligen Salz der allgemeinen Formel I umgesetzt werden.

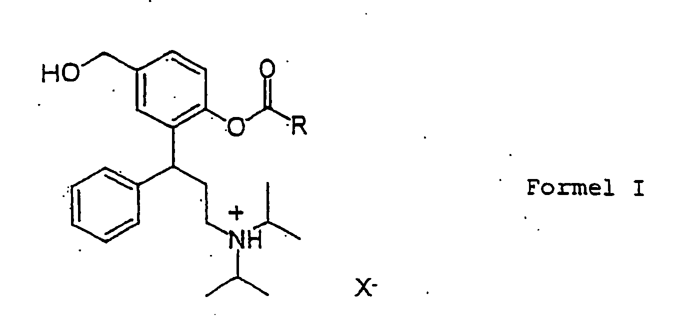

Es ist Aufgabe der vorliegenden Erfindung, eine hochreine, kristalline, stabile Verbindung aus neuartigen Derivaten von 3,3-Diphenylpropylaminen in Form eines ihrer Salze zur Verfügung zu stellen, die die erwähnten Nachteile vermeidet und sich besonders gut zum Einsatz in pharmazeutischtechnischen Formulierungen eignet und zu solchen verarbeiten lässt.

Weiterhin ist es Aufgabe der Erfindung, Zwischenprodukte zur Verfügung zu stellen, mit denen sich diese kristalline Verbindung chemo- und regioselektiv im hoher Ausbeute erhalten lässt.

Diese Aufgabe wurde gelöst durch die Verbindung *R*-(+)-2-(3-Diisopropylamino-1-phenylpropyl)-4-hydroxymethylphenylisobuttersäureester Hydrogenfumarat.

Die Verbindung der vorliegenden Erfindung ist das Salz
- *R*-(+)-2-(3-Diisopropylamino-1-phenylpropyl)-4-hydroxymethylphenylisobuttersäureester Hydrogenfumarat
   in kristalliner Form.

Weiterhin wird ein Verfahren zur Herstellung von R-konfigurierten Verbindungen der allgemeinen Formel 2 beschrieben, 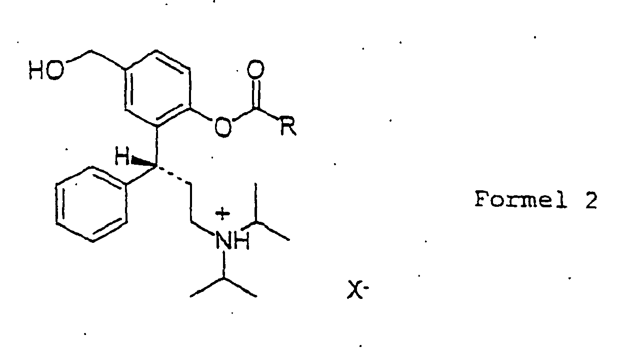 worin R für C₁-C₆ alkyl, steht und X⁻ für den Säurerest einer physiologisch verträglichen anorganischen oder organischen Säure steht, indem
a) eine Verbindung der Formel 3 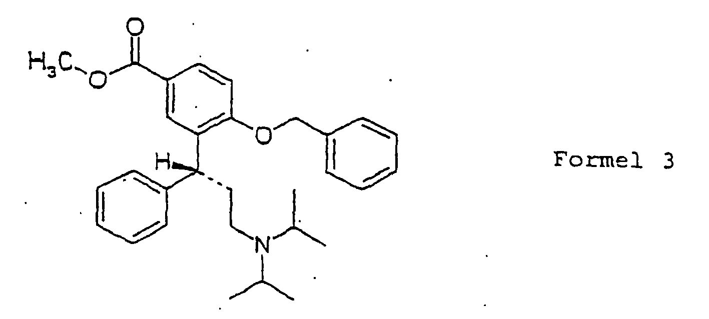 mit einem Hydrierungsmittel zur Bildung einer Verbindung der Formel 5 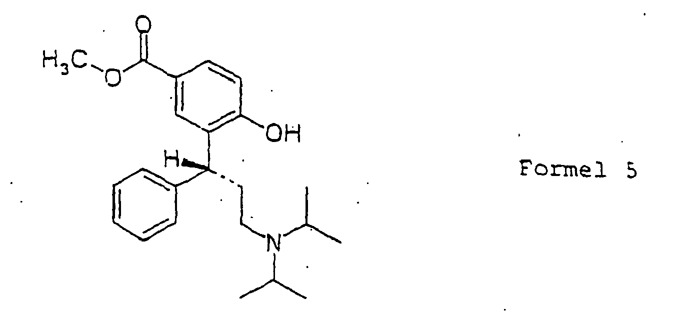 gespalten wird, worauf
b) die so erhaltene Verbindung der Formel 5 mit einem Reduktionsmittel umgesetzt wird, um eine Verbindung der Formel 6 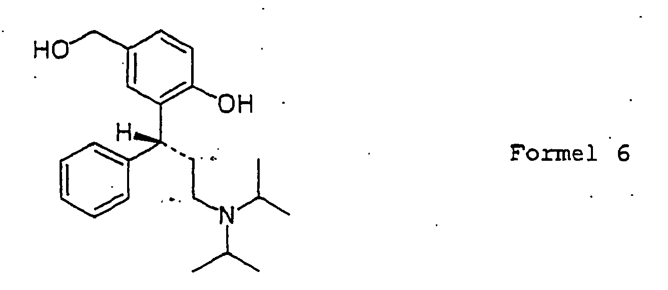 zu ergeben, die
c) mit einem Acylierungsmittel umgesetzt wird, um eine Verbindung der Formel 1 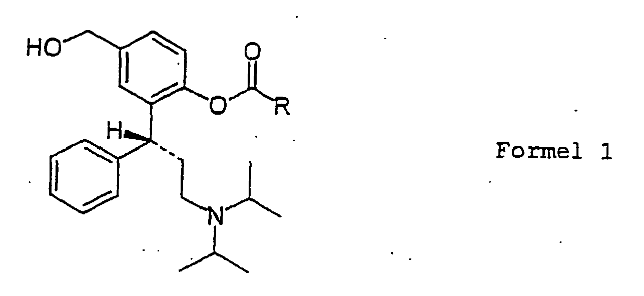 zu erhalten, worin R die oben genannte Bedeutung hat, die
d) mit einer physiologisch verträglichen anorganischen oder organischen Säure unter Bildung einer Verbindung der Formel 2 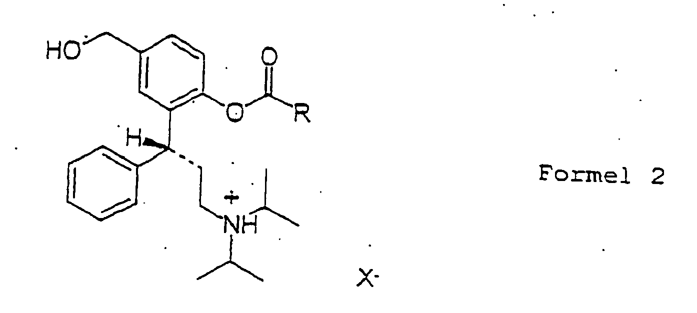
umgesetzt wird, worin R für C₁-C₆ alkyl, steht und X⁻ für den Säurerest einer physiologisch verträglichen anorganischen oder organischen Säure steht.

Zum Erhalt der Verbindung der allgemeinen Formel 2 wird die Säure Fumarsäure verwendet.

Je nach verwendetem Säurechlorid werden Verbindungen der allgemeinen Formel 1 erhalten, 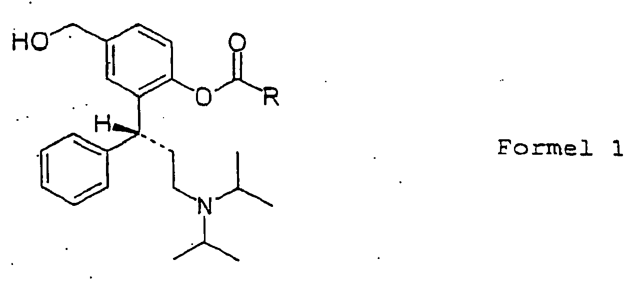 in der R die Bedeutung von C₁-C₆ alkyl, insbesondere Isopropyl hat.

Zum Erhalt der erfindungsgemäßen Verbindung ist die spezielle Reaktionsführung über besondere Zwischenstufen und individualisierbare Zwischenprodukte entscheidend.

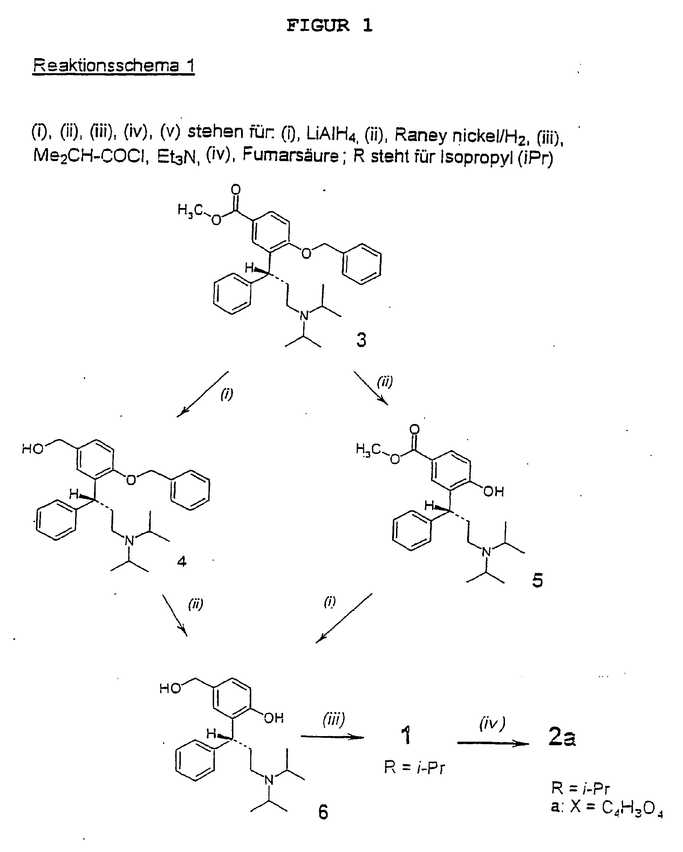

Dies wird anhand des Reaktionsschemas 1 (siehe Figur 1) erläutert, in dem Umsetzungen mit R-konfigurierten Verbindungen beschrieben werden.

Darin bedeuten:
- 3 =: *R*-(-)-4-Benzyloxy-3-(3-diisopropylamino-1-phenyl-propyl)-benzoesäure- methylester
- 4 =: *R*-(+)-[4-Benzyloxy-3-(3-diisopropylamino-1-phenyl-propyl) -phenyl] -methanol
- 5 =: *R*-(-)-3-(3-Diisopropylamino-phenyl-propyl)-4-hydroxybenzoesäuremethylester
- 6 =: *R-*(+)-2-(3-Diisopropylamino-1-phenylpropyl) -4-hydroxymethylpheaol
- 1 =: *R*-(+)-2=(3-Diisopropylamino-1-phenylpropyl)-4-hydroxymethylphenyl-isobuttersäureester
- 2a =: *R*-(+)-2-(3-Diisopropylamino-1-phenylpropyl)-4-hydroxymethylphenyl-isobuttersäureester Hydrogenfumarat

Entsprechend der in den Ausführungsbeispielen erläuterten Reaktionsführung wird die Vorstufe 3 (*R*-(-)-4-Benzyloxy-3-(3-diisopropylamino-1-phenyl-propyl)-benzoesäure-methylester) kristallin und rein dargestellt.

Vorstufe 3 wird nach üblichen Methoden - z.B. BBr₃, AlCl₃₋vorzugsweise jedoch mittels Wasserstoffgas über Raney-Nickel in Methanol als Lösungsmittel bei Raumtemperatur (RT) zu 5 (*R*- (-)-3-(3-Diisopropylamino-phenyl-propyl)-4-hydroxy-benzoesäuremethylester) gespalten. Dieses fällt in hochreiner, kristalliner Form (Schmp. 143.7 °C) an.

Schließlich wird 5 mit einem geeigneten Reduktionsmittel - z.B. NaBH₄/EtOH - vorzugsweise LiAlH₄ in einem inerten Lösungsmittel bei niedrigen Temperaturen (-78°C bis + 10°C) reduziert und die Verbindung 6 (*R*-(+)-2-(3-Diisopropylamino-1-phenylpropyl)-4-hydroxymethylphenol) erhalten. Die Verbindung 6 wird hochrein erhalten und kann aus einem geeigneten Lösungsmittel, wie beispielsweise Ethylacetat, kristallisiert werden. Das farblose feinkristalline Material besitzt einen Schmelzpunkt von 10.2 . 3 °C. Dies ist insofern überraschend, als die Verbindung 6 im Stand der Technik als amorpher Festkörper beschrieben wird.

Verbindung 6 wird nun in sehr guter Ausbeute und Regio- und Chemoselektivität, zu einem phenolischen Ester acyliert. Diese Reaktion wird bei RT oder niedrigen Temperaturen mit einem Äquivalent Säurechlorid in Gegenwart einer Base in geeigneten Lösungsmitteln ausgeführt. Geeignete Lösungsmittel sind Ethylacetat, Dichlormethan, Tetrahydrofuran, Acetonitril oder Toluol.

Bevorzugt wird die Reaktion mit Isobutyrylchlorid als Säurechlorid und Triethylamin als Base bei den oben angegebenen Temperaturen durchgeführt. Das dann erhaltene 1 (R-(+)-2-(3-Diisopropylamino-1-phenylpropyl)-4-hydroxymethylphenylisobuttersäureester) fällt in so hoher Reinheit an, daß mit Lösungen der Fumarsäure in geeigneten Lösungsmitteln spontane Kristallisation unter Bildung des Hydrogenfumarat-Salzes 2a einsetzt.

Dieses Salz zeigt einen scharfen Schmelzpunkt von 103°C, ist bei RT stabil, nicht hygroskopisch und schließt kein Kriställösemittel ein. Es läßt sich beliebig oft umkristallisieren.

### Verbindung der Formel 3

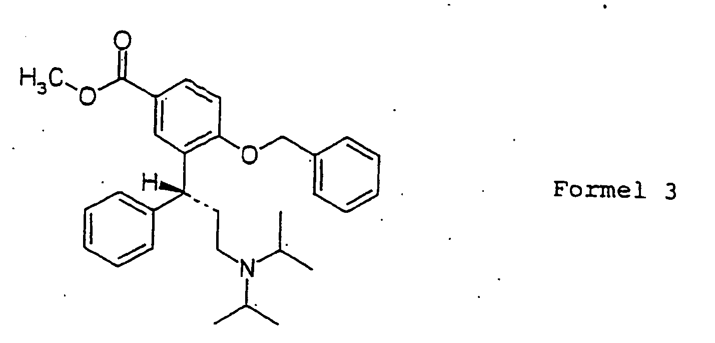

### Verbindung der Formel 5

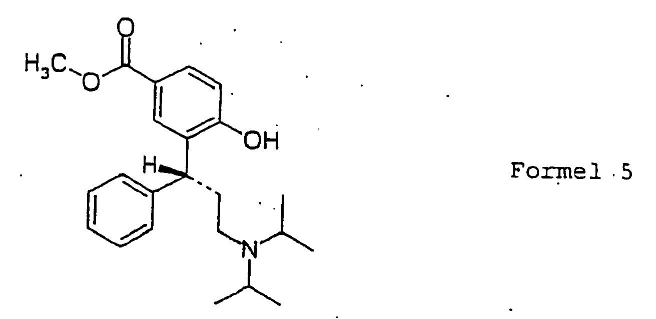

### Verbindung der Formel 6

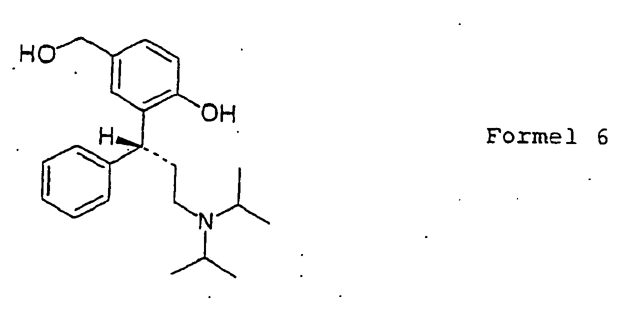

Die vorgenannten Verbindungen 3, 5 und 6 eignen sich besonders zur Verwendung als jeweils hochreines, kristallines, stabiles Zwischenprodukt bei der Herstellung von *R*-(+)-2-(3-Diisopropylamino-1-phenylpropyl)-4-hydroxymethylphenylisobuttersäureester Hydrogenfumarat.

Schließlich kann das Verfahren besonders vorteilhaft ausgeführt werden, indem eine Verbindung der allgemeinen Formel 6 (siehe Reaktionsschema 1) mit einem Äquivalent Isobutyrylchlorid in Gegenwart von Triethylamin unter Verwendung eines der jeweiligen Lösungsmittel Ethylacetat, Dichlormethan, Tetrahydrofuran, Acetonitril oder Toluol regio- und chemoselektiv zu *R*-(+)-2-(3-Diisopropylamino-1-phenylpropyl)-4-hydroxymethylphenylisobuttersäureester umgesetzt wird.

Verfahrensgernäß eignet sich besonders vorteilhaft *R*-(+)-2-(3-Diisopropylamino-1-phenylpropyl)-4-hydroxymethylphenylisobuttersäureester zur Umsetzung mit Fumarsäure unter Bildung des entsprechenden Salzes.

Die nachfolgenden Ausführungsbeispiele erläutern die Erfindung.

### Experimentelles

### I. Allgemeines

Alle Verbindungen wurden vollständig durch ¹H und ¹³C NMR-Spektroskopie charakterisiert (Bruker DPX 200). Die angeführten chemischen Verschiebungen in den ¹³C-NMR-Spektren (50 MHz, ppm Werte aufgeführt) beziehen sich auf die Lösungsmittelresonanzen von CDCl₃ (77. 10 ppm). ¹H NMR Daten (CDCl₃; 200 MHz, ppm) beziehen sich auf internes Tetramethylsilan).

Dünnschichtchromatographie (DC, R_{f} angegeben) wurde durchgeführt auf 5x10 cm E. Merck Kieselgelfolien (60F254), die Flecken wurden visualisiert durch Fluoreszenzlöschung oder Ansprühen mit alkalischer Kaliumpermanganatlösung.

Laufmittelsysteme waren: (1), n-Hexan / Aceton / Triethylamin (70/20/10, v/v-%); (2), Toluol / Aceton / Methanol / Essigsäure (70/5/20/5, v/v-%).

Die optischen Drehungen wurden bei einer Wellenlänge von 589.3 nm (Natrium D-Linie) vermessen, bei Raumtemperatur unter Verwendung des Lösungsmittels Ethanol (Gerät: Perkin Elmer Polarimeter Type 241),
Schmelzpunkte (Schmp., in °C) sind unkorrigiert und wurden am Gerät Mettler FP 1 bestimmt, bzw. Differentialthermoanalyse (DSC) am Perkin Elmer Modell DSC7, Auswertungssoftware "Pyris".

UV/VIS-Messungen wurden am Spektrophotometer Modell Lambda 7 (Perkin-Elmer) bei einer Schichtdicke von 1 cm durchgeführt. Angegeben ist die spezifische Absorption einer 1-%igen Lösung (A¹ %_{1 cm}).

IR-Spektren wurden an einem Perkin-Elmer FTIR Spektrometer Serie 1610 aufgezeichnet (Auflösung 4 cm⁻¹).

Gaschromatographie-Massenspektrometrie (GC-MS, m/z-Werte und relative Intensität bezogen auf das Basision (%)) wurde mit einem Finnigan TSQ 700 Triple Mass Spectrometer im positiv (P-CI) oder negativ (N-CI) chemische Ionisationsmeßbetrieb mit Methan oder Ammoniak als Reaktantgas bzw. über Elektronenstößionisation aufgenommen. Hydroxyverbindungen wurden als Trimethylsilylether-Derivate vermessen.

Gekoppelte Flüssigkeitschromatographie-Massenspektrometrie (LC-MS): Waters Integrety System, Thermabeam Mass Detector (EI, 70 eV), m/z-Werte und relative Intensität (%) werden über einen Massenbereich von 50-500 a.m.u. angegeben.

### II. Ausführungsbeispiele

Die in Klammern gesetzten arabischen Zahlen (3), (4), (5), (6) beziehen sich auf die identischen Bezeichnungen im Reaktionsschema 1.

### 1. Darstellung von R-(-)-4-Benzyloxy-3-(3-diisopropylamino-1-phenyl-propyl)-benzoesäuremethylester (3)

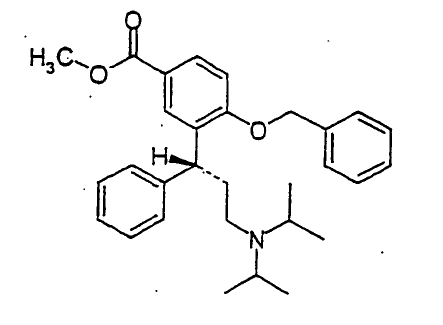

Eine Lösung von *R*-(-)-4-Benzyloxy-3-(3-diisopropylamino-T-phenyl-propyl)-benzoesäure Hydrochlorid (2.30 kg, 9.77 Mol) in 26.4 Liter Methanol und 0.25 Liter konzentrierter Schwefelsäure wird 16 Stunden unter Rückfluß erhitzt. Anschließend wird ein Drittel des Lösungsmittels abdestilliert, abgekühlt und unter Rühren mit 5 kg Eis und 2.5 Liter 25%-iger wässriger Kaliumcarbonatlösung versetzt. Der Ansatz wird erst mit 15 Liter, dann nochmals mit 5 Liter Dichlormethan extrahiert. Die organischen Phasen werden vereinigt und am Rotationsverdampfer zur Trockene eingeengt. Man erhält 1.99 kg (90.7 % der Theorie) hellgelbes Öl in ca. 90 % Reinheit (DC, NMR).
DC (1): 0.58
¹³C-NMR (CDCl₃) : 20.55, 20.65, 36.83, 41.84, 43.83, 51.82, 70.12, 111.09, 122.46, 125.28, 127.49, 128.02, 128.35, 128.50, 129.22, 129.49, 133.20, 136.39, 149.51; 159.87, 167.09.

### Umkristallisation

69.0 g öliges Rohprodukt werden in 150 ml siedendem Methanol gelöst. Nach dem Zusatz von 15 ml destilliertem Wasser wird bei 0°C belassen, wobei sich farblose Kristalle abscheiden. Diese werden abfiltriert, mit wenig kaltem Methanol gewaschen und im Vakuum getrocknet. Ausbeute: 41.8 g (60.6 % der Theorie) farblose Kristalle, Schmp. 89.8 °C; [I]_{D}²⁰ = - 30.7 (c = 1.0, Ethanol).

### 2. Darstellung von . R-(+)-[4-Benzyloxy-3-(3-diisopropylamino-1-phenyl-propyl)-phenyl]-methanol (4)

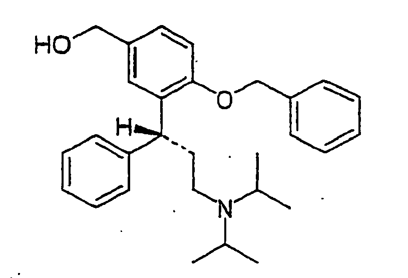

Rohprodukt (3) (28 g) wird in 230 ml absolutem Diethylether gelöst und unter Rühren in eine Suspension von 1.8 g Lithiumaluminiumhydrid in Diethylether (140 ml) getropft. Nach 18 Stunden Rühren bei Raumtemperatur werden tropfenweise 4.7 ml Wasser zugesetzt. Die organische Phase wird abgetrennt, mit wasserfreiem Natriumsulfat getrocknet, filtriert und am Rotationsverdampfer zur Trockene eingeengt. Man erhält 26 g (98.9 % der Theorie) *R*-(+)-[4-Benzyloxy-3-(3-diisopropylamino-1-phenyl-propyl)-phenyl]-methanol (4) als farbloses Öl. DC (2): 0.32; [I]_{D}²⁰ = + 6.3 (c = 1.0, Ethanol) .
¹³C-NMR (CDCl₃) : 20.53, 20.61, 36.87, 41.65, 44.14, 48.82, 65.12, 70.09, 111.80, 125.77, 125.97, 126.94, 127.55, 128.08, 128.37, 128.44, 133.27, 134.05, 134.27, 137.21, 144.84.

### 3. Darstellung von R-(-)-3-(3-Diisopropylamino-phenyl-propyl)-4-hydroxybenzoesäuremethylester (5)

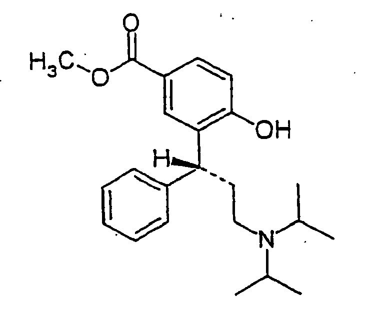

Zu einer gerührten Suspension von 5 g Raney-Nickel (mit Wasser, dann mit Methanol gewaschen) in 200 ml Methanol werden 10 g (21.8 mmol) *R*-(-)-4-Benzyloxy-3-(3-diisopropylamino-1-phenyl-propyl)-benzoesäuremethylester (3) zugesetzt. Nach kurzem Erwärmen, um alles (3) vollständig zu lösen, wird die Apparatur unter eine Atmosphäre von Wasserstoffgas gesetzt. Nach drei Stunden Rühren bei Normaldruck und Raumtemperatur zeigt die Dünnschichtchromatographie vollständige Umsetzung. Der Ansatz wird mit Stickstoffgas gespült und nach Zusatz von etwas Aktivkohle filtriert. Nach dem Einengen der methanolischen Lösung am Rotationsverdampfer verbleiben 6.0 g (75 % der Theorie) *R*-(-)-3-(3-Diisopropylaminophenyl-propyl)-4-hydroxy-benzoesäuremethylester (5) in Form farbloser Kristalle in einer Reinheit von 99.6 % (HPLC).
Schmp. 143.7°C; DSC 144.7°C
[I]_{D}²⁰ = - 26.6 (c = 0.93, Ethanol).
¹³C-NMR (CDCl₃) : 18.74, 19.21, 19.62, 33.12, 39.68, 42.36, 48.64, 51.42, 117.99, 120.32, 126.23, 127.81, 128.85, 129.39, 130.26, 132.21, 144.06, 162.43, 167.35.

### 4. Darstellung von R-(+)-2-(3-Diisopropylamino-1-phenylpropyl)-4-hydroxymethyl-phenol (6)

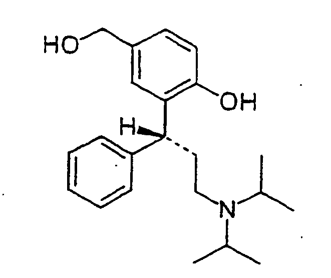

### a) Ausgehend von der Zwischenstufe (4), R-(+)-[4-Benzyloxy-3-(3-diisopropylamino-1-phenyl-propyl)-phenyl] -methanol

*R*-(+)-[4-Benzyloxy-3-(3-diisopropylamino-1-phenyl-propyl)-phenyl]-methanol (19.7 g, 45.7 mmol) werden in 220 ml Methanol gelöst und mit Raney-Nickel (5 g) versetzt. Die Apparatur wird mit Wasserstoffgas gespült und der Ansatz zwei Tage bei Raumtemperatur gerührt. Nach dem Zusatz von weiteren 5 g Raney-Nickel wird zwei weitere Tage bei Raumtemperatur unter Wasserstoffgasatmosphäre gerührt, vom Katalysator abfiltriert und das Filtrat am Rotationsverdampfer zur Trockene eingeengt. Der ölige, blaßgelbe Rückstand wird in 100 ml Diethylether gelöst, zweimal mit je 100 ml Wasser gewaschen, über Natriumsulfat getrocknet, filtriert und zur Trockene eingeengt. Man erhält 14.1 g (90.4 % der Theorie) *R*-(+)-2-(3-Diisopropylamino-1-phenylpropyl)-9-hydroxymeth ylphenol in Form eines cremefarbenen, amorphen Festkörpers. Umkristallisation siehe unten c).

### b) Ausgehend von der Zwischenstufe (5); R-(-)-3-(3-Diisopropylamino-phenyl-propyl)-4-hydroxy-benzoesäuremethylester

Eine Lösung von 370 mg (1.0 mmol) *R*-(-)-3-(3-Diisopropylamino-phenyl-propyl)-4-hydroxy-benzoesäuremethylester in 20 ml wasserfreiem Tetrahydrofuran wird langsam und bei Raumtemperatur zu einer gerührten Mischung von trockenem Tetrahydrofuran (10 ml) und einer IM Lösung von Lithiumaluminiumhydrid in Tetrahydrofuran (3 ml) (unter Stickstoffschutzgasatmosphäre) getropft. Überschüssiges Hydrid wird durch tropfenweisen Zusatz einer gesättigten Natriumcarbonatlösung zersetzt. Nach dem Abtrennen der organischen Phase wird diese am Rotationsverdampfer eingeengt und anschließend im Hochvakuum getrocknet. Es werden 274 mg (74 % der Theorie) blaßgelbes Öl erhalten, das sich langsam zu einer amorphen Masse verfestigt.

### c) Umkristallisation:

Rohprodukt 6 (1.0 g) wird in Ethylacetat gelöst und am Rotatiorisverdampfer erneut eingeengt. Das so von Fremdlösernitteln (Diethylether bzw. Tetrahydrofuran, s.o.) befreite Diol wird unter leichtem Erwärmen mit 1.5 ml Ethylacetat versetzt. Man rührt, bis eine klare Lösung entstanden ist, läßt auf Raumtemperatur abkühlen und setzt einige Impfkristalle zu. Letztere werden gewonnen, indem rohes 6 über HPLC gereinigt wird, die Hauptfraktion aufgefangen wird, eingeengt und der Rückstand mehrere Stunden im Hochvakuum getrocknet wird. Nachdem deutliche Kristallisation eingetreten ist, beläßt man bei - 10 °C. Die Kristalle werden noch in der Kälte abgesaugt und im Vakuum getrocknet. Man erhält farblose Kristalle in 84 % Ausbeute.
Schmp. 102.3 °C
DC (1): 0.57
[I] _{D}²⁰ = + 21.3 (c = 1.0, Ethanol).
¹³C-NMR (CDCl₃) : 19.58, 19.96, 33.30, 39.52, 42.10, 48.00, 65.40, 118.58, 126.31, 126.57, 127.16, 127.54, 128.57, 132.63, 132.83, 144.55, 155.52.

### 5. Darstellung von R-(+)-2-(3-Diisopropylamino-1-phenylpropyl)-4-hydroxymethylphenylisobuttersäureester (1)

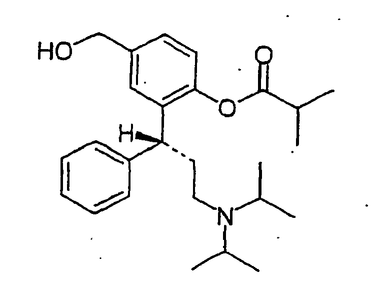

Eine Lösung von *R*-(+)-2-(3-Diisopropylamino-1-phenylpropyl)-4-hydroxymethylphenol (6) (65.0 g, 190.3 mmol) und Triethylamin (20.4 g, 201.7 mmol) in 750 ml Dichlormethan wird unter Rühren und Kühlung (-5 °C) mit einer Lösung von Isobuttersäurechlorid (23.4 g, 201.7 mmol) in 250 ml Dichlormethan versetzt. Nach der Zugabe wird noch 15 Min. bei 0 °C, dann 30 Min. bei Raumtemperatur gerührt und nacheinander mit Wasser (250 ml) und 5%-iger wässriger Natriumhydrogencarbonat-Lösung gewaschen. Die organische Phase wird abgetrennt und am Rotationsverdampfer zur Trockene eingeengt. Der Ester *R*-(+)-2-(3-Diisopropylamino-1-phenylpropyl)-4-hydroxymethyl-phenylisobuttersäureester wird als farbloses; viskoses Öl erhalten; Ausbeute: 77.1 g (98.4 % der Theorie).
DC (1): 0.26; [I]_{D}²² = + 2.7 (c = 1.0, Ethanol).
¹³C-NMR (CDCl₃):19.01, 19.95, 20.59, 21.12, 34.28, 36.89, 41.88, 42.32, 43.90, 48.78, 64.68, 122.57, 125.59, 126.16, 126. 86, 127. 96, 128.54, 136. 88, 138.82, 143.92, 147.90, 175.96.

### 6. Darstellung von R-(+)-2-(3-Diisopropylamino-1-phenylpropyl)-4-hydroxymethylphenylisobuttersäureester Hydrogenfumarat

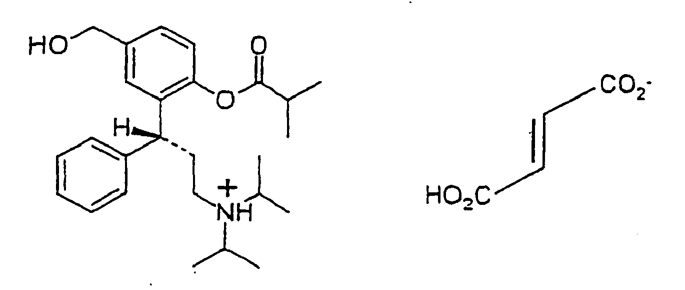

Eine Lösung von 41.87 g (102 mmol) *R*-(+)-2-(3-Diisopropylamino-1-phenylpropyl) -4-hydroxymethylphenylisobuttersäureester in 90 ml 2-Butanon wird unter Erwärmen mit Fumarsäure (11.81 g, 102 mmol) versetzt. Nach dem Lösen der Säure wird langsam unter Rühren Cyclohexan (20-30 ml) bis zum Einsetzen einer Trübung zugesetzt. Man beläßt den farblosen, homogenen Ansatz zunächst 18 Stunden bei Raumtemperatur, dann mehrere Stunden bei 0 °C. Die ausgefallenen farblosen Kristalle werden abgesaugt, mit wenig Cyclohexan/2-Butanon (90:10, Vol.-%) gewaschen und im Vakuum bei 30 °C getrocknet. Man erhält 44.6 g (83.1 % der Theorie) des Hydrogenfumarat-Salzes des R-(+)-2-(3-Diisopropylamino-1-phenylpropyl)-4-hydroxymethylphenylisobuttersäureester in Form farbloser Plättchen.
Schmp. 98.8 °C, eine zweite Kristallisation aus dem gleichen Lösungsmittelgemisch ergibt das Produkt mit einem Schmp. von 103 °C.
[I]_{D}²⁰ = + 6.0 (c = 1.0, Ethanol).
Elementaranalyse: Berechnet für C₃₀H₄₁NO₇ (Molgewicht 527.66) C 68.29 %, H 7.83 %, N 2.65 %, O 21.2 %; gefunden C 68.29 %, H 7.90 %, N 2.72 %, O 21.0 %.
UV/VIS bei Σ in nm (A ^{1%}_{1 cm}): 191 (1306), 193 (1305), 200 (1143), 220 (456).
IR: 3380, 2978, 2939, 2878, 2692, 2514, 1756, 1702, 1680, 1618, 1496, 1468, 1226, 1040, 1019, 806,
¹H-NMR (CDCl₃) : 1.198, 1.285, 1.287 (CH₃) ; 2. 541 (CHC=O) 3.589 (NCH); 4.585 (CH₂OH); 6.832 (=CH, Fumarat); 6.84-7.62 (Aryl, = CH)
¹³C-NMR (CDCl₃):17.79, 18.95, 19.16 (CH₃) ; 31.63 (CHCH₂); 34.09 (CH-C=O); 41.87 (CHCH₂); 45.83 (NCH₂); 54.29 (NCH); 63.78 (OCH₂); 122.23, 126.48, 126.77, 127.56, 140.46, 140.52, 142.35, 147.54 (Aryl CH); 135.54 (=CH, Fumarat); 170.48 (C=O, Fumarat); 175.62 (i-Pr-C=O).
MS im Direkteinlaß, m/z (%) : 411 (1), 396 (9), 380 (1), 223 (2), 165 (2), 114 (100), 98 (4), 91 (3), 84 (3), 72 (10), 56 (7).

## Patentansprüche

1. Verwendung einer Verbindung ausgewählt aus den folgenden Formeln 3, 5 und 6, 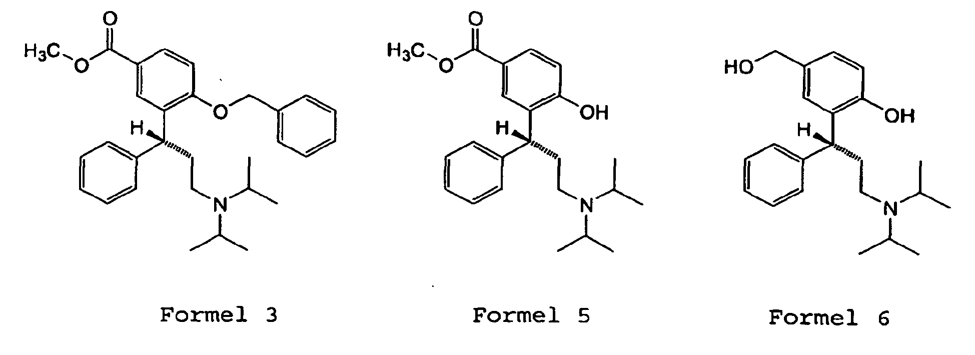 als Zwischenprodukt bei der Herstellung von kristallinem *R-*(+)-2-(3-Diisopropylamino-1-phenylpropyl)-4-hydroxymethylphenylisobuttersäureester Hydrogenfumarat.

2. *R*-(+)-2-(3-Diisopropylamino-1-phenylpropyl)-4-hydroxymethyl-phenylisobuttersäureester Hydrogenfumarat in kristalliner Form.

## Claims

1. Use of a compound selected from the following formulae 3, 5 and 6 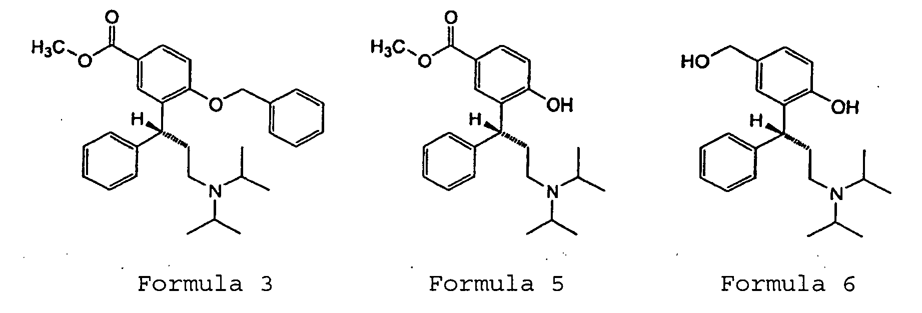 as an intermediate in the preparation of crystalline *R*-(+)-2-(3-diisopropylamino-1-phenylpropyl)-4-hydroxymethylphenylisobutyrate hydrogen fumarate.

2. *R*-(+)-2-(3-Diisopropylamino-1*-*phenylpropyl)-4-hydroxymethylphenylisobutryate hydrogen fumarate in crystalline form.

## Revendications

1. Utilisation d'un composé choisi parmi les formules 3, 5 et 6 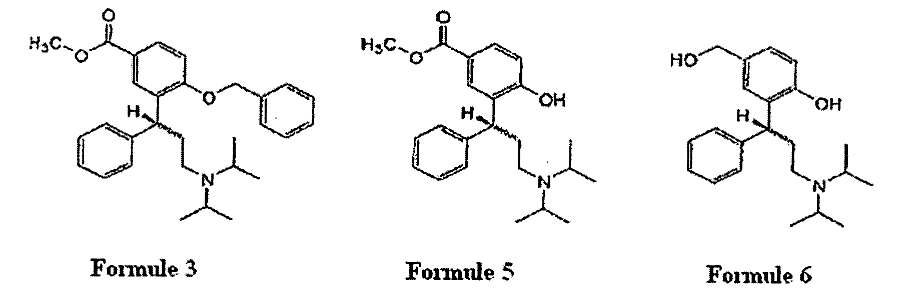 en tant que produit intermédiaire pour la production d'ester hydrogénofumarique de l'acide R-(+)-2-(3-diisopropylamino-1-phénylpropyl)-4-hydroxyméthyl-phénylisobutyrique cristalin.

2. Ester hydrogénofumarique de l'acide R-(+)-2-(3-diisopropylamino-1-phénylpropyl)-4-hydroxyméthyl-phénylisobutyrique dans une forme cristalline.
